# EUROPEAN PATENT SPECIFICATION

(11) **EP 1 301 238 B1**
(45) Date of publication and mention of the grant of the patent: **22.09.2004**
(21) Application number: 01962862.7
(22) Date of filing: 18.07.2001
(51) Int. Cl.: A61M 37/00

(54) **Needles coated with vaccine**
Mit Impfstoff beschichtete nadeln
Aiguilles enduites d'un vaccin

(30) Priority: 21.07.2000 GB 0017999
(43) Date of publication of application: 16.04.2003
(73) Proprietor: SMITHKLINE BEECHAM BIOLOGICALS S.A., 1330 Rixensart (BE); SMITHKLINE BEECHAM PLC, Brentford, Middlesex TW8 9GS (GB)
(72) Inventor: DALTON, Colin Clive, GlaxoSmithKline Biologicals, B-1330 Rixensart (BE); EASEMAN, Richard Lewis, GlaxoSmithKline, Brentford, Middlesex TW8 9EP (GB); GARCON, Nathalie, GlaxoSmithKline Biologicals, B-1330 Rixensart (BE)
(74) Representative: Easeman, Richard Lewis
(86) International application number: PCT/EP2001/008339
(87) International publication number: WO 2002/007813

(56) References cited:
- EP-A- 0 737 472
- WO-A-97/03718
- WO-A-99/64580
- US-A- 2 619 962
- US-A- 5 990 194

## Description

The present invention relates to efficient devices for administration of pharmaceutical agents into the skin of the human body. In particular the present invention provides devices for vaccination into the skin. The present invention provides a pharmaceutical agent delivery device having skin-piercing portion comprising a solid reservoir medium containing the pharmaceutical agent, wherein the reservoir medium is coated onto the skin piercing portion. Alternatively, the skin piercing portion may consist of the solid pharmaceutical agent reservoir medium. The devices of the present invention are storage stable, and only substantially release the pharmaceutical after penetration of the skin piercing portion into the skin. In a preferred embodiment there is provided a microneedle device coated externally with the solid reservoir medium that releases the pharmaceutical agent directly into the skin after piercing the stratum corneum. The pharmaceutical delivery devices are proportioned such that agent is delivered into defined layers of the skin, and preferred delivery devices comprise skin-piercing portions that deliver the pharmaceutical agent into the epithelium or the dermis. Preferred reservoir media comprise sugars, and in particular stabilising sugars that form a glass such as lactose, raffinose, trehalose or sucrose. Furthermore, vaccine delivery devices for administration of vaccines into the skin are provided, methods of their manufacture, and their use in medicine.

The skin represents a significant barrier to external agents. A summary of human skin is provided in Dorland's Illustrated Medical Dictionary, 28^{th} Edition. Starting from the external layers, working inwards, the skin comprises the epithelium comprising the stratum corneum, the viable epithelium, and underlying the epithelium is the dermis. The epithelium consists of five layers: Stratum corneum, Stratum lucidium, Stratum granulosum, Stratum spinosum, and Stratum basale. The *epithelium* (including all five layers) is the outermost non-vascular layer of the skin, and varies between 0.07 and 0.12 mm thick (70-120 µm). The epithelium is populated with keratinocytes, a cell that produces keratin and constitutes 95% of the dedicated epidermal cells. The other 5% of cells are melanocytes. The underlying dermis is normally found within a range of 0.3 to about 3 mm beneath the surface of the stratum corneum, and contains sweat glands, hair follicles, nerve endings and blood vessels.

The stratum corneum dominates the skin permeability barrier and consists of a few dozen horny, keratinised epithelium layers. The narrow interstices between the dead or dying keratinocytes in this region are filled with crystalline lipid multilamellae. These efficiently seal the interstices between the skin or body interior and the surroundings by providing a hydrophobic barrier to entry by hydrophylic molecules. The stratum corneum being in the range of 30-70 µm thick.

Langerhans cells are found throughout the basal granular layer of the epithelium (stratum spinosum and stratum granulosum, (Small Animal Dermatology - Third Edition, Muller - Kirk - Scott, Ed: Saunders (1983)) and are considered to play an important role in the immune system's initial defence against invading organisms. This layer of the skin therefore represents a suitable target zone for certain types of vaccine.

Conventional modes for administration of pharmaceutical agents into or across the skin, most commonly by hypodermic needle and syringe, are associated with numerous disadvantages. Such disadvantages include pain, the requirement for trained professionals to administer the agent, and also the risk of needle-stick injuries to the administrator with the accompanying risk of infection with a blood born disease. As such, there is a need to improve the method of administration of all types of pharmaceutical into or through the skin.

A number of alternative approaches have been described in order to overcome the problems of administering agent across the stratum corneum, including various designs of skin patches. Examples of skin patches which deliver agent through the skin without physically penetrating the stratum corneum layer include that described in WO 98/20734 and WO 99/43350. Other approaches where the skin is not physically punctured include electrotransport, or iontophoretic devices where the passage of agent is enhanced by the application of an electrical current into the skin.

Many such devices are described in the literature (examples of which include US 6,083,190; US 6.057,374; US 5,995,869; US 5,622,530). Potential disadvantages of these types of non-penetration patches include the induction of significant sensitisation and discomfort during administration of the agent, and very poor uptake of antigen across the intact stratum corneum.

Other patches involving physical disruption or penetration of the skin have been described. Devices comprising liquid or solid reservoirs containing agent and a metal microblade patch have been described wherein the microblades physically cut through the stratum corneum to create pathways through which the agent can enter the epithelium. Such devices are described in WO 97/48440, WO 97/48442, WO 98/28037, WO 99/29298, WO 99/29364, WO 99/29365, WO 00/05339, WO 00/05166, and WO 00/16833. Other devices involving puncturing of the skin include US 5,279,544, US 5,250,023 and US 3,964,482. Some of the disadvantages of these types of devices arise from generally poor rates of uptake of agent over the time of administration, despite the microblades penetrating the stratum corneum. The poor rates of uptake, results in long 'dwell times' during which the microblades are in contact with the skin. For conventional vaccination purposes, dwell times of longer than about fifteen to 30 minutes are relatively undesirable as they would prolong the period that the vaccinee needs to be monitored to check for possible side effects such as anaphylactic shock. In addition, many of the previously described products need to be transported and/or stored in refrigerated space. The larger volume of these products compared to vials means that fewer doses can be stored in the end-users refrigerators and making logistics more complicated and expensive.

Solid dosage forms comprising a pharmaceutical agents and a stabilising polyol, such as a sugar wherein the dosage forms are in the form of powders and trocars are described in WO 96/03978.

The present invention provides for improved devices that are stable during storage, and are capable of administering and releasing agent efficiently into or through the skin. The invention is achieved by providing pharmaceutical delivery devices having at least one skin-piercing member that is loaded with a biodegradable reservoir medium containing the agent to be delivered, the loaded skin-piercing member, such as a needle, being long enough and sharp enough to pierce the stratum corneum of the skin. Once the pharmaceutical agent delivery device has been administered to the surface of the skin, and the coated skin-piercing member or microneedle has pierced through the stratum corneum, the reservoir medium biodegrades thereby releasing the agent into the skin underlying the stratum corneum.

In a preferred form of the present invention there is provided a delivery device having at least one skin-piercing portion and a solid reservoir medium containing the pharmaceutical agent, wherein the reservoir medium is coated externally onto the skin piercing portion. Alternatively, the skin piercing portion may consist of the solid pharmaceutical agent reservoir medium.

The devices of the present invention may be used to administer any agent to a patient, which is desired to be administered in a short time frame in a painless manner without the dangers and fear often associated with conventional needles and devices. Examples of such agents include those agents that are required to be delivered daily, such as insulin, but also those agents that are required less frequently such as vaccines or genes for correction of genetic disorders.

Vaccine delivery devices form a preferred aspect of the present invention. In such applications the agent to be delivered is an antigen or antigens and may comprise micro-organisms or viruses (live, attenuated or killed) or gene or nucleic acid vectors (eg adenovirus, retrovirus), an antigen derived from a pathogen (such as a sub-unit, particle, virus like particle, protein, peptide, polysaccharide or nucleic acid) or may be a self antigen in the case of a cancer vaccine or other self antigen associated with a non-infectious, non-cancer chronic disorder such as allergy. The agent may be antigen or nucleic acid alone or it may also comprise an adjuvant or other stimulant to improve and/or direct the immune response, and may also further comprise pharmaceutically acceptable excipient(s). The vaccine coated devices may be used for prophylactic or therapeutic vaccination and for priming and/or boosting the immune response. In cases of therapeutic vaccination where it is necessary to break tolerance then vaccine coated patches may be used as part of a specific regimen such as prime boost. Certain embodiments of the device described herein also have the significant advantage of being stored at room temperature thus reducing logistic costs and releasing valuable refrigerator space for other products.

The delivery devices of the present invention can be used for a wide variety of pharmaceutical agents that can not easily be administered using conventional non-penetration patches such (as hydrophilic molecules) in the absence of penetration enhancers.

The skin piercing protrusions which may be coated with reservoir medium to form preferred delivery devices of the present invention may be made of almost any material which can be used to create a protrusion that is strong enough to pierce the stratum corneum and which is safe for the purpose, for example the protrusions may be made of a metal, such as pharmaceutical grade stainless steel, gold or titanium or other such metal used in prostheses, alloys of these or other metals; ceramics, semiconductors, silicon, polymers, plastics, glasses or composites.

The patch generally comprise a backing plate from which depend a plurality of piercing protrusions such as microneedles or microblades. The piercing protrusions themselves may take many forms, and may be solid or hollow, and as such may be in the form of a solid needle or blade (such as the microblade aspects and designs described in McAllister *et al., Annu. Rev. Biomed. Eng.,* 2000, 2, 289-313; Henry *et al.,* Journal of Pharmaceutical Sciences, 1998, 87, 8, 922-925; Kaushik *et al., Anesth. Analg.,* 2001, 92, 502-504; McAllister *et al., Proceed. Int'l. Symp. Control. Rel. Bioact. Mater.,* 26, (1999), Controlled Release Society, Inc., 192-193; WO 99/64580; WO 97/48440; WO 97/48442; WO 98/28037; WO 99/29364; WO 99/29365; US 5,879,326, the designs of all of these documents, and the methods of manufacture of the microblade arrays being incorporated herein by reference). Alternatively the piercing protrusions may be in the form of a microneedle having a hollow central bore. In this last embodiment, the central bore may extend through the needle to form a channel communicating with both sides of the microneedle member (EP 0 796 128 B1). Solid microneedles and microblades are preferred.

The length of the skin-piercing member is typically between 1 µm to 1 mm, preferably between 50µm and 600µm, and more preferably between 100 and 400µm. The length of the skin-piercing member may be selected according to the site chosen for targeting delivery of the agent, namely, preferably, the dermis and most preferably the epidermis. The skin-piercing members of the devices of the present invention may be take the form of, and be manufactured by the methods described in US 5,879,326, WO 97/48440, WO 97/48442, WO 98/28037, WO 99/29298, WO 99/29364, WO 99/29365, WO 99/64580, WO 00/05339, WO 00/05166, or WO 00/16833; or McAllister *et al., Annu. Rev. Biomed. Eng.,* 2000, 2, 289-313; Henry *et al.,* Journal of Pharmaceutical Sciences, 1998, 87, 8, 922-925; *Kaushik et al., Anesth. Analg.,* 2001, 92, 502-504; McAllister *et al., Proceed. Int'l. Symp. Control. Rel. Bioact, Mater.,* 26, (1999), Controlled Release Society, Inc., 192-193.

The most preferred microblade devices to be coated with the pharmaceutical agent reservoir medium to form devices of the present invention are described in WO 99 48440 and *Henry et al.,* Journal of Pharmaceutical Sciences, 1998, 87, 8, 922-925.

The devices of the present invention preferably comprise a plurality of skin-piercing members, preferably up to 1000 members per device, more preferably up to 500 skin-piercing members per device.

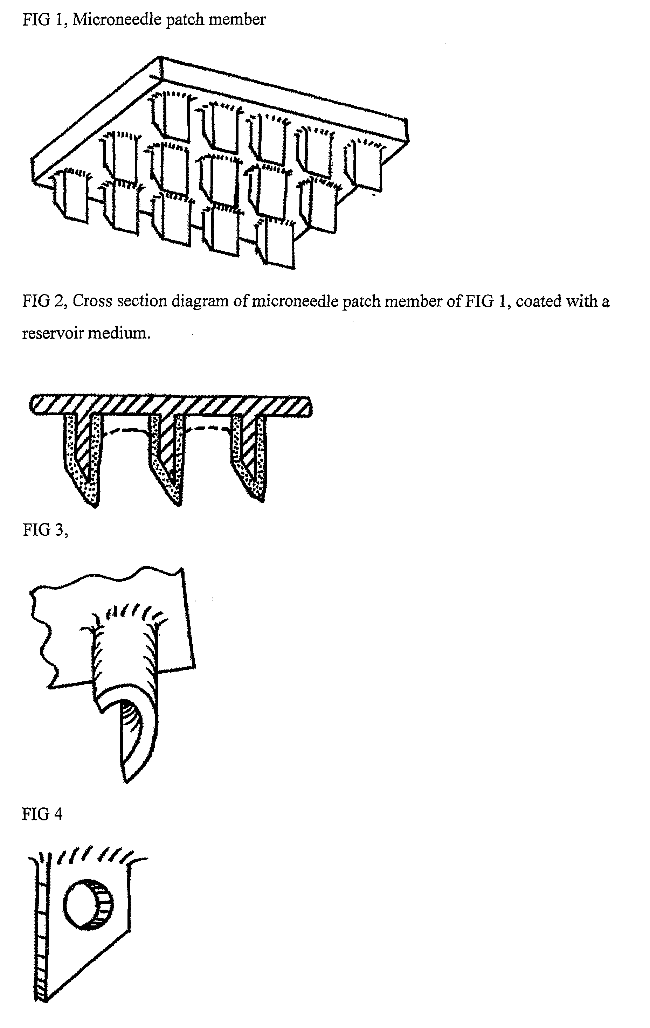

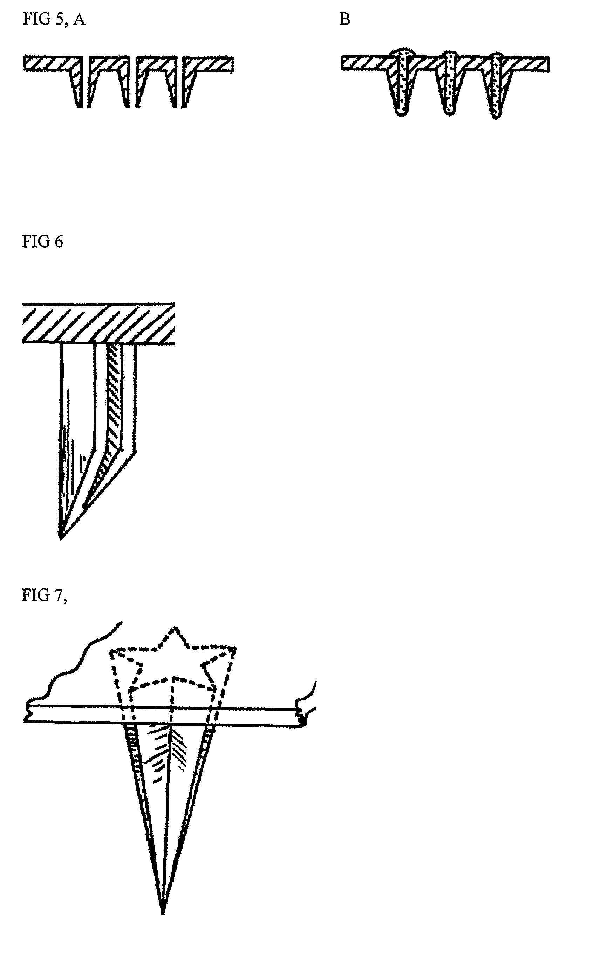

Where the piercing protrusion is solid, it may flat (termed microblade, see FIG 1) or may have a circular or polgonal cross section (see FIG 5). The protrusions can have straight or tapered shafts and may be flat or circular, or other polygonal shape, in cross section. For example, the microblades may have a curved blade (FIG 3) or be formed into a V-section groove (FIG 6). Alternatively the protrusions may have more complex shapes to enhance adherence and fluid dynamics such as a five pointed star shown in FIG 7.

The skin-piercing members may be integral with the backing plate or may be attached thereto. In the case where the protrusions may be attached to the plate, the piercing protrusion may be formed of the reservoir medium. Such devices may be made by formed by drawing or extruding a molten reservoir medium containing the agent into fine points. For instance, molten reservoir medium could be cast directly onto a backing plate through a multipore head, where the hot extrudate cools and sticks to the plate. When you draw back the extrudate a series of pointed ends is formed.

As a general feature of any piercing protrusion shape, in order to improve reservoir adherence after coating, the surface of the protrusion may be textured. For example, the surface may be coarse grained, rippled or ribbed. In addition, solid microblades may further comprise holes (see FIG 4), such that the reservoir may dry therein and create a reservoir tie, to hold the reservoir onto the blade more securely. In certain embodiments, including highly soluble and friable lyophilised formulations, it is preferred that the friable reservoir may be entirely held within such holes thereby protected from breakage during puncture of the skin.

In an alternative embodiment the piercing protrusions may be separable from the base member. For example, in the embodiment where the piercing protrusions (or at least the tips thereof) is the reservoir itself, after penetration of the skin the piercing protrusions separates from the base support thus allowing the patch to be removed from the skin, whilst leaving the reservoir behind in the skin. The separation of the reservoir from the backing plate may be by physical shearing or by biodegradation of part of the needles adjacent the backing plate.

One embodiment of this may be to cast the microprotrusion tips out of a relatively poorly soluble disaccharide reservoir medium (containing a dispersion of the agent to be delivered) followed by casting the remaining portion of the microprotrusion and backing plate out of a relatively easily soluble material. Once inserted into the skin, the relatively easily soluble microprotrusion shaft would degrade away, thereby allowing the patch to be removed from the skin, whilst leaving the tips within the skin. The tips, remaining in the skin can then slowly release the agent by slower biodegradation.

Accordingly, in a preferred embodiment of the present invention there is provided a skin patch for delivery of pharmaceutical agents or vaccines comprising an array of microblades or microneedles coated with a solid biodegradable reservoir medium containing the pharmaceutical agent or vaccine.

The biodegradable agent reservoir may be any made from any medium that fulfils the function required for the present invention. The reservoir must be capable of adhering to the microprotrusion to a sufficient extent that the reservoir remains physically stable and attached during prolonged storage, and also remains substantially intact during the administration procedure when the coated microprotrusion pierce the stratum corneum. The reservoir must also be capable of holding or containing a suspension or solution of agent to be delivered in any dry or partially dry form, which is released into the skin during biodegradation of the reservoir medium.

Biodegradation of the medium in the sense of the present invention means that the reservoir medium changes state, such that changes from its non-releasing to its releasing states whereby the agent enters into the skin. The release of the active agent may involve one or more physical and/or chemical processes such as hydration, diffusion, phase transition, crystallisation, dissolution, enzymatic reaction and/or chemical reaction. Depending on the choice of reservoir medium, biodegradation can be induced by one or more of the following: water, body fluids, humidity, body temperature, enzymes, catalysts and/or reactants. The change of the reservoir medium may therefore be induced by hydration, and warming associated with the higher humidity and temperature of the skin. The reservoir medium may then degrade by dissolution and/or swelling and/or change phase (crystalline or amorphous), thereby disintegrating or merely increase the permeation of the medium.

Preferably the medium dissolves, and is metabolised or expelled or excreted from the body, but the reservoir may alternatively remain attached to the skin-piercing member to be removed from the skin when the device is removed. Release of the agent by dissolution of the reservoir medium is preferred.

Examples of suitable reservoir media include, but are not restricted to, polyols such as sugars, polysaccharides, substituted polyols such as hydrophobically derivatised carbohydrates, amino acids, biodegradable polymers or co-polymers such as poly(hydroxy acid)s, polyahhydrides, poly(ortho)esters, polyurethanes, poly(butyric acid)s, poly(valeric acid)s, and poly(lactide-co-caprolactone)s, or polylactide co-glycolide. The coating of the microblades may be in the amorphous or crystalline state and may also be partially amorphous and partially crystalline.

Particularly preferred reservoir media are those that stabilise the agent to be delivered over the period of storage. For example, antigen or agent dissolved or dispersed in a polyol glass or simply dried in a polyol are storage stable over prolonged periods of time (US 5,098,893, US 6,071,428; WO 98/16205; WO 96/05809; WO 96/03978; US 4,891,319; US 5,621,094; WO 96/33744). Such polyols form the preferred set of reservoir media.

Preferred polyols include sugars, including mono, di, tri, or oligo saccharides and their corresponding sugar alcohols. Suitable sugars for use in the present invention are well known in the art and include, trehalose, sucrose, lactose, fructose, galactose, mannose, maltulose, iso-maltulose and lactulose, maltose, or dextrose and sugar alcohols of the aforementioned such as mannitol, lactitol and maltitol. Sucrose, Lactose, Raffinose and Trehalose are preferred.

It is preferred that the reservoir medium forms an amorphous glass upon drying. The glass reservoir may have any glass transition temperature, but preferably it has a glass transition temperature that both stabilises the pharmaceutical agent during storage and also facilitates rapid release of the agent after insertion of the reservoir into the skin. Accordingly, the glass transition temperature is greater than 30-40°C, but most preferably is around body temperature (such as, but not limited to 37-50°C).

The preferred reservoir media used to cost the skin-piercing members of the devices are those that release the pharmaceutical agent over a short period of time. The preferred reservoir formulations release substantially all of the agent within 5 minutes, more preferably within 2 minutes, more preferably within 1 minute, and most preferably within 30 seconds of insertion into the skin. Such fast releasing reservoirs can be achieved, for example, by thin coatings of amorphous glass reservoirs, particularly fast dissolving/swelling glassy reservoirs having low glass transition temperatures. It will be clear to the man skilled in the art that a low glass transition temperature can be achieved by selecting the appropriate glass forming sugar, and/or increasing humidity and/or ionic strength of the glass. Additionally, increased speed of dissolution of glass reservoirs may also be achieved by warming the device before or during application to the skin.

Other suitable excipients which may be included in the formulation include buffers, amino acids, phase change inhibitors ('crystal poisoners') which may be added to prevent phase change of the coating during procesing or storage or inhibitors to prevent deleterious chemical reactions during processing or storage such Maillard reaction inhibitors like amino acids.

Accordingly, in a preferred embodiment of the present invention there is provided a skin patch for delivery of vaccines comprising an array of microblades or microneedles coated with a glassy sugar reservoir medium containing the vaccine.

The reservoir medium is preferably of a solid or extremely viscous solution, which may itself be smooth or textured. For example, the medium may be solid, crystalline, amorphous/glassy, solid solution, solid suspension, porous, smooth, rough, or rugose.

The formulations comprising the agent to be delivered and biodegradable reservoir medium are preferably mixed in aqueous solution and then dried onto the microprotrusion member or the formulation could be melted and then applied to the microprotrusion member. A preferred process for coating the skin-piercing members comprises making an aqueous solution of vaccine antigen and water soluble polyol (such as trehalose), followed by coating the solution onto the microblades by dipping the member into the solution one or more times followed by drying at ambient temperature or lyophilisation to give a porous coating (repeating the process in part or whole to build up the depth of coating required, see FIG 2 - for a coated microblade (dotted area being reservoir medium - dashed lines showing that the reservoir medium may cover the entire undersurface of the microblade member)). In this process it is preferred that the initial solution of water soluble polyol or sugar is viscous, such as the viscosity achieved from 40% sugar.

In an embodiment where the microneedles have hollow central bores (FIG 5A) or the microblades are curved or have a V-section (FIGs 3 and 6) once the blade is dipped into the liquid medium, the liquid solution will rise up and fill the bore or internal spaces by capilliary action (for a microneedle having a central bore after loading with reservoir medium see FIG 5B).

Alternatively, minute picolitre volumes of solution or melted formulation may be sprayed onto individual blades by technology commonly used in the art of bubble-jet printers, followed by drying. An alternative method would be to prepare microspheres or microparticles or powders of amorphous formulation containing polyol such as sugar, using techniques known in the art (such as spray drying or spray freeze drying or drying and grinding) and by controlling the moisture content to achieve a relatively low glass transition temperature (for example 30°C), followed by spraying or dipping to bring the micropheres or microparticles or powders into contact with a microprotrusion member heated to a temperature above that of the glass transition temperature of the microsphere (for example 45°C). The coated particles would then melt and adhere to the microprotrusion member and then dry or the coated microblade member would be further dried (to remove residual moisture content) thereby increasing the glass transition temperature of the reservoir medium suitable for storage.

Alternatively, the microneedle member may be coated using a freeze coating technique. For example, the temperature of the microneedle member may be lowered below that of the freezing point of water (for example by dipping in liquid nitrogen) and then aqueous solutions of the reservoir medium and agent my be sprayed onto the cold microneedles, or the microblade may be dipped into the solution of agent. In this way the agent and reservoir medium rapidly adheres to the microneedle member, which can then be sublimed by lyophilisation, or evaporated at higher temperatures, to dry the reservoir coating.

Another method to coat the microneedle members is to dip the microneedles in a solvent, such as water (optionally comprising a surfactant to ensure good contact) then dipping wetted blades in a powdered form of the reservoir medium which is soluble in the solvent, followed by drying to remove the solvent.

In a preferred embodiment of the invention there is provided a process for coating a microblade with a viscous solution of reservoir forming medium which is sufficiently fluid to allow sterile filtration through a 220 nm pore membrane. Accordingly there is provided a vaccine formulation comprising antigen in a filterable viscous sugar solution formulation. Preferred examples of such filterable viscous sugar solutions are solutions of between about 20 to about 50 % sugar (weight/volume of the final vaccine formulation prior to drying). More preferably the viscous filterable sugar solutions are in the range of about 30% to about 45% sugar, and most preferable are about 40% (weight sugar/volume of the final vaccine formulation prior to drying). In this context the most preferred sugar solutions comprise sucrose, raffinose, trehalose or lactose.

In the embodiment where the microblades comprise integral holes for dosing, strings of microblades (like a hacksaw blade) comprising individual blades like the one shown in figure 4, may be filled with reservoir and dried, before assembly into a patch. One such device assembled from many strings of blades is described in WO 99/29364. Alternatively, devices such as those described in WO 97/48440 may comprise integral holes, which may be filled whilst the blades are still in the plane of the etched base plate, followed by the blades being punched into the perpendicular alignment with the reservoir medium in situ.

Using these techniques each skin piercing member may be loaded with relatively high amounts of pharmaceutical agent. Each piercing member preferably being loaded with up to 500 ng or pharmaceutical or antigen, more preferably up to 1 µg of pharmaceutical or antigen and more preferably up to 5µg of pharmaceutical or antigen.

Preferably the vaccine formulations of the present invention contain an antigen or antigenic composition capable of eliciting an immune response against a human pathogen, which antigen or antigenic composition is derived from HIV-1, (such as tat, nef, gp120 or gp 160), human herpes viruses, such as gD or derivatives thereof or Immediate Early protein such as ICP27 from HSV1 or HSV2, cytomegalovirus ((esp Human)(such as gB or derivatives thereof), Rotavirus (including live-attenuated viruses), Epstein Barr virus (such as gp350 or derivatives thereof), Varicella Zoster Virus (such as gpI, II and IE63), or from a hepatitis virus such as hepatitis B virus (for example Hepatitis B Surface antigen or a derivative thereof), hepatitis A virus, hepatitis C virus and hepatitis E virus, or from other viral pathogens, such as paramyxoviruses: Respiratory Syncytial virus (such as F and G proteins or derivatives thereof), parainfluenza virus, measles virus, mumps virus, human papilloma viruses (for example HPV6, 11, 16, 18, ..), flaviviruses (e.g. Yellow Fever Virus, Dengue Virus, Tick-borne encephalitis virus, Japanese Encephalitis Virus) or Influenza virus (whole live or inactivated virus, split influenza virus, grown in eggs or MDCK cells, or Vero cells or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as *Neisseria spp,* including *N. gonorrhea* and *N. meningitidis* (for example capsular polysaccharides and conjugates thereof, transferrin-binding proteins, lactoferrin binding proteins, PilC, adhesins); *S. pyogenes* (for example M proteins or fragments thereof, C5A protease, lipoteichoic acids), *S. agalactiae, S. mutans; H. ducreyi; Moraxella spp, including Mcatarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M. tuberculosis* (for example ESAT6, Antigen 85A, -B or -C), *M bovis, M leprae, M avium, M paratuberculosis, M smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E. coli,* enteropathogenic *E*. *coli* (for example shiga toxin-like toxin or derivatives thereof); *Vibrio spp,* including *V. cholera* (for example cholera toxin or derivatives thereof); *Shigella spp,* including S. *sonnei, S. dysenteriae, S. flexnerii; Yersinia spp,* including *Y*. enterocolitica (for example a Yop protein) , *Y. pestis, Y. pseudotuberculosis; Campylobacter spp,* including C. *jejuni* (for example toxins, adhesins and invasins) and *C. coli; Salmonella spp,* including *S*. *typhi, S. paratyphi, S. choleraesuis, S. enteritidis; Listeria spp.,* including *L. monocytogenes; Helicobacter spp,* including *H.* pylori (for example urease, catalase, vacuolating toxin); *Pseudomonas spp,* including *P. aeruginosa; Staphylococcus spp.,* including *S. aureus, S. epidermidis; Enterococcus spp.,* including *E. faecalis, E. faecium; Clostridium spp.,* including *C.* tetani (for example tetanus toxin and derivative thereof), *C. botulinum* (for example botulinum toxin and derivative thereof), *C*. *difficile* (for example clostridium toxins A or B and derivatives thereof); *Bacillus spp.,* including *B. anthracis* (for example botulinum toxin and derivatives thereof); *Corynebacterium spp.,* including *C. diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii; Ehrlichia spp.,* including *E. equi* and the agent of the Human Granulocytic Ehrlichiosis; *Rickettsia spp,* including *R. rickettsii; Chlamydia spp., including C. trachomatis* (for example MOMP, heparin-binding proteins), *C. pneumoniae* (for example MOMP, heparin-binding proteins), *C. psittaci; Leptospira spp.,* including *L. interrogans; Treponema spp.,* including *T. pallidum* (for example the rare outer membrane proteins), *T. denticola, T. hyodysenteriae;* or derived from parasites such as *Plasmodium spp.,* including *P. falciparum; Toxoplasma spp.,* including *T. gondii (for example SAG2, SAG3, Tg34); Entamoeba spp.,* including *E*. *histolytica; Babesia spp.,* including *B. microti; Trypanosoma spp.,* including *T. cruzi; Giardia spp.,* including *G. lamblia; Leshmania spp.,* including *L. major; Pneumocystis spp.,* including *P. carinii; Trichomonas spp.,* including *T. vaginalis; Schisostoma spp.,* including *S. mansoni,* or derived from yeast such as *Candida spp.,* including *C. albicans; Cryptococcus spp.,* including *C. neoformans.*

Preferred bacterial vaccines comprise antigens derived from *Streptococcus spp,* including *S. pneumoniae* (for example capsular polysaccharides and conjugates thereof, PsaA, PspA, streptolysin, choline-binding proteins)and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy varients or fusion proteins thereof. Other preferred bacterial vaccines comprise antigens derived from *Morexella Catarrhalis* (including outer membrane vesicles thereof, and OMP106 (WO97/41731)) and from *Neisseria mengitidis B* (including outer membrane vesicles thereof, and NspA (WO 96/29412).

Derivatives of Hepatitis B Surface antigen are well known in the art and include, inter alia, those PreS1, PreS2 S antigens set forth described in European Patent applications EP-A-414 374; EP-A-0304 578, and EP 198-474. In one preferred aspect the vaccine formulation of the invention comprises the HIV-1 antigen, gp120, especially when expressed in CHO cells. In a further embodiment, the vaccine formulation of the invention comprises gD2t as hereinabove defined.

In a preferred embodiment of the present invention vaccines containing the claimed adjuvant comprise antigen derived from the Human Papilloma Virus (HPV) considered to be responsible for genital warts, (HPV 6 or HPV 11 and others), and the HPV viruses responsible for cervical cancer (HPV16, HPV18 and others).

Particularly preferred forms of genital wart prophylactic, or therapeutic, vaccine comprise L1 particles or capsomers, and fusion proteins comprising one or more antigens selected from the HPV 6 and HPV 11 proteins E6, E7, L1, and L2.

The most preferred forms of fusion protein are: L2E7 as disclosed in WO 96/26277, and proteinD(1/3)-E7 disclosed in GB 9717953.5 (PCT/EP98/05285):

A preferred HPV cervical infection or cancer, prophylaxis or therapeutic vaccine, composition may comprise HPV 16 or 18 antigens. For example, L1 or L2 antigen monomers, or L1 or L2 antigens presented together as a virus like particle (VLP) or the L1 alone protein presented alone in a VLP or capsomer structure. Such antigens, virus like particles and capsomer are per se known. See for example WO94/00152, WO94/20137, WO94/05792, and WO93/02184.

Additional early proteins may be included alone or as fusion proteins such as preferably E7, E2 or E5 for example; particularly preferred embodiments of this includes a VLP comprising L1E7 fusion proteins (WO 96/11272).

Particularly preferred HPV 16 antigens comprise the early proteins E6 or E7 in fusion with a protein D carrier to form Protein D - E6 or E7 fusions from HPV 16, or combinations thereof; or combinations of E6 or E7 with L2 (WO 96/26277).

Alternatively the HPV 16 or 18 early proteins E6 and E7, may be presented in a single molecule, preferably a Protein D- E6/E7 fusion. Such vaccine may optionally contain either or both E6 and E7 proteins from HPV 18, preferably in the form of a Protein D - E6 or Protein D - E7 fusion protein or Protein D E6/E7 fusion protein. The vaccine of the present invention may additionally comprise antigens from other HPV strains, preferably from strains HPV 6, 11, 31, 33, or 45.

Vaccines of the present invention further comprise antigens derived from parasites that cause Malaria. For example, preferred antigens from *Plasmodia falciparum* include RTS,S and TRAP. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. It's full structure is disclosed in the International Patent Application No. PCT/EP92/02591, published under Number WO 93/10152 claiming priority from UK patent application No.9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is a Malaria vaccine wherein the antigenic preparation comprises a combination of the RTS,S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.

The formulations may also contain an anti-tumour antigen and be useful for the immunotherapeutic treatment cancers. For example, the adjuvant formulation finds utility with tumour rejection antigens such as those for prostrate, breast, colorectal, lung, pancreatic, renal or melanoma cancers. Exemplary antigens include MAGE 1 and MAGE 3 or other MAGE antigens for the treatment of melanoma, PRAME, BAGE or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma. Other Tumor-Specific antigens are suitable for use with adjuvant of the present invention and include, but are not restricted to Prostate specific antigen (PSA) or Her-2/neu, KSA (GA733), MUC-1 and carcinoembryonic antigen (CEA). Accordingly in one aspect of the present invention there is provided a vaccine comprising an adjuvant composition according to the invention and a tumour rejection antigen.

Additionally said antigen may be a self peptide hormone such as whole length Gonadotrophin hormone releasing hormone (GnRH, WO 95/20600), a short 10 amino acid long peptide, in the treatment of many cancers, or in immunocastration.

It is foreseen that compositions of the present invention will be used to formulate vaccines containing antigens derived from *Borrelia sp..* For example, antigens may include nucleic acid, pathogen derived antigen or antigenic preparations, recombinantly produced protein or peptides, and chimeric fusion proteins. In particular the antigen is OspA. The OspA may be a full mature protein in a lipidated form virtue of the host cell (E.Coli) termed (Lipo-OspA) or a non-lipidated derivative. Such non-lipidated derivatives include the non-lipidated NS1-OspA fusion protein which has the first 81 N-terminal amino acids of the non-structural protein (NS1) of the influenza virus, and the complete OspA protein, and another, MDP-OspA is a non-lipidated form of OspA carrying 3 additional N-terminal amino acids. Vaccines of the present invention may be used for the prophylaxis or therapy of allergy. Such vaccines would comprise allergen specific (for example. Der pl) and allergen non-specific antigens (for example peptides derived from human IgE, including but not restricted to the stanworth decapeptide (EP 0 477 231 B1)).

It is foreseen that compositions of the present invention will be used to formulate vaccines containing antigens derived from a wide variety of sources. For example, antigens may include human, bacterial, or viral nucleic acid, pathogen derived antigen or antigenic preparations, tumour derived antigen or antigenic preparations, host-derived antigens, including GnRH and IgE peptides, recombinantly produced protein or peptides, and chimeric fusion proteins.

Additionally the compositions of the present invention can include nucleic acids either in naked form or incorporated in a suitable vector such as adenovirus or retrovirus to aid incorporation of the nucleic acids into the cells of the skin after application. Applications of this embodiment include DNA vaccines and gene therapy products.

Plasmid based delivery of genes, particularly for immunisation or gene therapy purposes is known. For example, administration of naked DNA by injection into mouse muscle is outlined in WO90/11092 Johnston et al WO 91/07487 describe methods of transferring a gene to veterbrate cells, by the use of microprojectiles that have been coated with a polynucleotide encoding a gene of interest, and accelerating the microparticles such that the microparticles can penetrate the target cell.

DNA vaccines usually consist of a bacterial plasmid vector into which is inserted a strong viral promoter, the gene of interest which encodes for an antigenic peptide and a polyadenylation/transcriptional termination sequences. The gene of interest may encode a full protein or simply an antigenic peptide sequence relating to the pathogen, tumour or other agent which is intended to be protected against. The plasmid can be grown in bacteria, such as for example *E.coli* and then isolated and prepared in an appropriate medium, depending upon the intended route of administration, before being administered to the host. Following administration the plasmid is taken up by cells of the host where the encoded protein or peptide is produced. The plasmid vector will preferably be made without an origin of replication which is functional in eukaryotic cells, in order to prevent plasmid replication in the mammalian host and integration within chromosomal DNA of the animal concerned. Information in relation to DNA vaccination is provided in Donnelly *et al* "DNA vaccines" *Ann. Rev Immunol.* 1997 15: 617-648.

In an embodiment of the invention, a polynucleotide is administered/delivered as "naked" DNA, for example as described in Ulmer et al., *Science* 259:17.45-1749, 1993 and reviewed by Cohen, *Science 259*:1691-1692, 1993. The uptake of naked DNA may be increased by coating the DNA onto inert metallic beads, such as gold, or biodegradable beads, which are efficiently transported into the cells; or by using other well known transfection facilitating agents, such as Calcium Phosphate.

DNA may be administered in conjunction with a carrier such as, for example, liposomes, and everything being entrapped in the reservoir medium. Typically such liposomes are cationic, for example imidazolium derivatives (WO95/14380), guanidine derivatives (WO95/14381), phosphatidyl choline derivatives (WO95/35301) piperazine derivatives (WO95/14651) and biguanide derivatives.

Vaccines of the present invention, may advantageously also include an adjuvant. Suitable adjuvants for vaccines of the present invention comprise those adjuvants that are capable of enhancing the antibody responses against the IgE peptide immunogen. Adjuvants are well known in the art (Vaccine Design - The Subunit and Adjuvant Approach, 1995, Pharmaceutical Biotechnology, Volume 6, Eds. Powell, M.F., and Newman, M.J., Plenum Press, New York and London, ISBN 0-306-44867-X). Preferred adjuvants for use with immunogens of the present invention include aluminium or calcium salts (hydroxide or phosphate).

Preferred adjuvants for use with immunogens of the present invention include: aluminium or calcium salts (hydroxide or phosphate), oil in water emulsions (WO 95/17210, EP 0 399 843), or particulate carriers such as liposomes (WO 96/33739). Immunologically active saponin fractions (e.g. Quil A) having adjuvant activity derived from the bark of the South American tree Quillaja Saponaria Molina are particularly preferred. Derivatives of Quil A, for example QS21 (an HPLC purified fraction derivative of Quil A), and the method of its production is disclosed in US Patent No. 5,057,540. Amongst QS21 (known as QA21) other fractions such as QA17 are also disclosed. 3 De-O-acylated monophosphoryl lipid A is a well known adjuvant manufactured by Ribi Immunochem, Montana. It can be prepared by the methods taught in GB 2122204B. A preferred form of 3 De-O-acylated monophosphoryl lipid A is in the form of an emulsion having a small particle size less than 0.2µm in diameter (EP 0 689 454 B1).

Adjuvants also include, but are not limited to, muramyl dipeptide and saponins such as Quil A, bacterial lipopolysaccharides such as 3D-MPL (3-O-deacylated monophosphoryl lipid A), or TDM. As a further exemplary alternative, the protein can be encapsulated within microparticles such as liposomes, or in non-particulate suspensions or aqueous solutions ofpolyoxyethylene ether of general formula (I)

HO(CH₂CH₂O)ₙ-A-R

wherein, n is 1-50, A is a bond or -C(O)-, R is C₁₋₅₀ alkyl or Phenyl C₁₋₅₀ alkyl (WO 99/52549).
Particularly preferred adjuvants are combinations of 3D-MPL and QS21 (EP 0 671 948 B1), oil in water emulsions comprising 3D-MPL and QS21 (WO 95/17210, PCT/EP98/05714), 3D-MPL formulated with other carriers (EP 0 689 454 B1), or QS21 formulated in cholesterol containing liposomes (WO 96/33739), or immunostimulatory oligonucleotides (WO 96/02555).

Examples of suitable pharmaceutically acceptable excipients include water, phosphate buffered saline, isotonic buffer solutions.

Also adjuvant preparations comprising an admixture of either polyoxyethylene castor oil or caprylic/capric acid glycerides, with polyoxyethylene sorbitan monoesters, and an antigen, are capable of inducing systemic immune responses after topical administration to a mucosal membrane (WO 9417827). This patent application discloses the combination of TWEEN20™ (polyoxyethylene sorbitan monoester) and Imwitor742™ (caprylic/capric acid glycerides), or a combination of TWEEN20™ and polyoxyethylene castor oil is able to enhance the systemic immune response following intranasal immunisation. Novasomes (US 5,147,725) are paucilamenar vesicular structures comprising Polyoxyethylene ethers and cholesterol encapsulate the antigen and are capable of adjuvanting the immune response to antigens after systemic administration.

Surfactants have also been formulated in such a way as to form non-ionic surfactant vesicles (commonly known as neosomes, WO 95/09651).

Other adjuvants which are known to enhance both mucosal and systemic immunological responses include the bacterial enterotoxins derived from Vibrio Cholerae and Eschericia Coli (namely cholera toxin (CT), and heat-labile enterotoxin (LT) respectively). CT and LT are heterodimers consisting of a pentameric ring of β-subunits, cradling a toxic A subunit. Their structure and biological activity are disclosed in Clements and Finklestein, 1979, Infection and Immunity, 24:760-769; Clements et al., 1980, Infection and Immunity, 24:91-97. Recently a non-toxic derivative of LT has been developed which lacks the proteolytic site required to enable the non-toxic form of LT to be "switched on" into its toxic form, once released from the cell. This form of LT (termed mLT(R192G)) is rendered insuceptible to proteolytic cleavage by a substitution of the amino acid arginine with glycine at position 192, and has been shown to have a greatly reduced toxicity whilst retaining its potent adjuvant activity. mLT(RI92G) is, therefore, termed a proteolytic site mutant. Methods for the manufacture of mLT(R192G) are disclosed in the patent application WO 96/06627. Other mutant forms of LT include the active site mutants such as mLT(A69G) which contain a substitution of an glycine for an alanine in position 69 of the LTA sequence. The use of mLT(R192G) as a mucosal vaccine is described in patent application WO 96/06627. Such adjuvants may be advantageously combined with the non-ionic surfactants of the present invention.

Other adjuvants or immunostimulants include the oligonucleotide adjuvant system containing an unmethylated CpG dinucleotide (as described in WO 96/02555). A particularly preferred immunostimulant is CpG immunostimulatory oligonucleotide, which formulations are potent in the induction and boosting of immune responses in larger animals. Preferred oligonucleotides have the following sequences: The sequences preferably contain all phosphorothioate modified internucleotide linkages. OLIGO 1: TCC ATG ACG TTC CTG ACG TT (SEQ ID NO. 1) OLIGO 2: TCT CCC AGC GTG CGC CAT (SEQ ID NO. 2) OLIGO 3: ACC GAT GAC GTC GCC GGT GAC GGC ACC ACG (SEQ ID NO. 3) The CpG oligonucleotides utilised in the present invention may be synthesized by any method known in the art (eg EP 468520). Conveniently, such oligonucleotides may be synthesized utilising an automated synthesizer.

Alternatively polyoxyethylene ethers or esters may be combined with vaccine vehicles composed of chitosan or other polycationic polymers, polylactide and polylactide-co-glycolide particles, particles composed of polysaccharides or chemically modified polysaccharides, cholesterol-free liposomes and lipid-based particles, oil in water emulsions (WO 95/17210), particles composed of glycerol monoesters, etc.

It is an intention of the present invention to administer agent or vaccine into the skin rapidly and with high yield of administration. This may be even further enhanced by a number of means, comprising the use of highly soluble carbohydrates as the reservoir medium, and also by agitating and/or heating the microneedle member during administration.

The amount of protein in each vaccine dose is selected as an amount which induces an immunoprotective response without significant adverse side effects in typical vaccinees. Such amount will vary depending upon which specific immunogen is employed and how it is presented. Generally, it is expected that each dose will comprise 1-1000 µg of protein, preferably 1-500 µg, more preferably 1-100 µg, of which 1 to 50µg is the most preferable range. An optimal amount for a particular vaccine can be ascertained by standard studies involving observation of appropriate immune responses in subjects. Following an initial vaccination, subjects may receive one or several booster immunisations adequately spaced.

The formulations mentioned may be used for both prophylactic and therapeutic purposes.

The present invention is exemplified by, but not limited to, the following examples.

### Example 1, Sugar coating formulations for vaccines

A Hepatitis B vaccine was produced, and formulated in 4 different sugars prior to coating onto a metallic needle. The Hepatitis vaccine (HepB) consisted of recombinant Hepatitis B surface antigen particles (as described in *Harford et al.,* 1983, *Develop. Biol. Standard,* 54, 125; and Gregg *et al,* 1987, Biotechnology, 5, 479; EP 0 266 846A and EP 0 299 108A). In brief, metal needles were dipped inside a solution of HepB and sugar, and then lyophilised. Coating of HepB onto the needles was confirmed by application of the dry coated needles to a gel.

### Materials

Lactose solution 15.75 %
Sucrose solution 15.75 %
Sucrose solution at 80% in water prepared from sucrose
Raffinose solution 15.75 % (D(+)-raffinose pentahydrate, Fluka 411308/1 12900) Trehalose solution 15.75 %
EPI 2001B60CB096
HepB Purified bulk
Needles: needle n° 8, article n° 121 292 from Prym, 52220 Stolberg, Germany Gel:Novex Pre-cast gel 4-20% Tris-Glyvine gel 1.0 mm. X 15 well

### Coating and lyophilisation of needles with HepB at 178 µg/ml in 4 different sugar formulations.

Hep B at 178 µg/ml was formulated in 4 different sugars at 3.15% (w/v).
Needles are fixed on a standard rubber stopper used in the lyophilisation vials. Needles are coated by plunging (2,5 cm deep) them once into the liquid Hep B formulations. Needles and rubber stopper are placed in a regular lyophilisation vial, and submitted to a standard lyophilisation cycle. After lyophilisation, the vials were closed by pushing completely the stopper on the vial, so that the coated needles are kept in a closed vial during storage.

| Lactose | Sucrose | Raffinose | Trehalose | PO₄ | NaCl | HbsAg |
|---|---|---|---|---|---|---|
| 3.15% | - | - | - | 2 mM | 30 mM | 178 µg/ml |
| - | 3.15% | - | - | 2 mM | 30 mM | 178 µg/ml |
| - | - | 3.15% | - | 2 mM | 30 mM | 178 µg/ml |
| - | - | - | 3.15% | 2 mM | 30 mM | 178 µg/ml |

### Analysis & SDS-PAGE conditions offormulated Hep B (before lyophilisation)

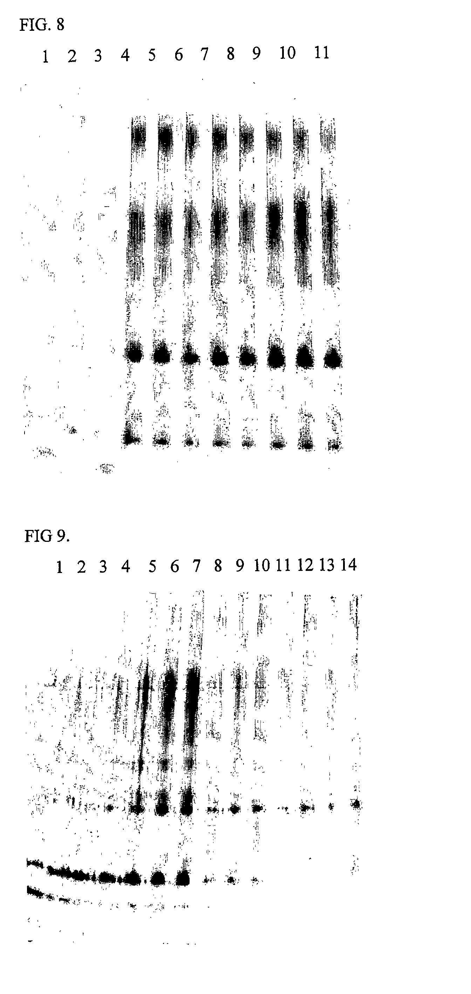

Samples of each formulation are applied on gel, as control, without any reducing treatment. 3 µl of each solution (representing 0.5 µg of protein) are loaded into a 4-20 % tris-glycine Novex gel. After electrophoresis silver stain is applied. The results are shown in FIG 8. The gel lanes correspond to: 1. MW marker (Biolabs); 2. Purified Bulk HepB; 3. MW marker (Biolabs); 4 and 5. Hep B coated in Lactose; 6 and 7. HepB coated in Sucrose; 8 and 9. HepB coated in Raffinose; 10 and 11. HepB coated in Trehalose.

### Analysis & SDS-PAGE conditions of coated needles (after lyophilisation)

Dry coated needles of each formulation are applied directly on gel by inserting them briefly (2 cm deep) inside the gel. No reducing treatment is applied. After electrophoresis, silver stain is applied. The results are shown in FIG. 9, the lanes correspond to: 1. MW marker (Biolabs); 2. Purified Bulk HepB; 3, 4 and 5, Needle lyophilised with formulation Lactose; 6, 7 and 8. Needle lyophilised with formulation Sucrose; 9, 10 and 11. Needle lyophilised with formulation Raffinose; 12, 13 and 14, Needle lyophilised with formulation Trehalose.

### Conclusions

No degradation between liquid formulation (see Fig n° 2) and lyophilised formulation on needle (see Fig n°3). Both liquid and lyophilised samples give similar pictures on the gel. No difference between lactose, sucrose, raffinose, or trehalose. Presence of protein on each needle.

### Example 2, Release kinetic test

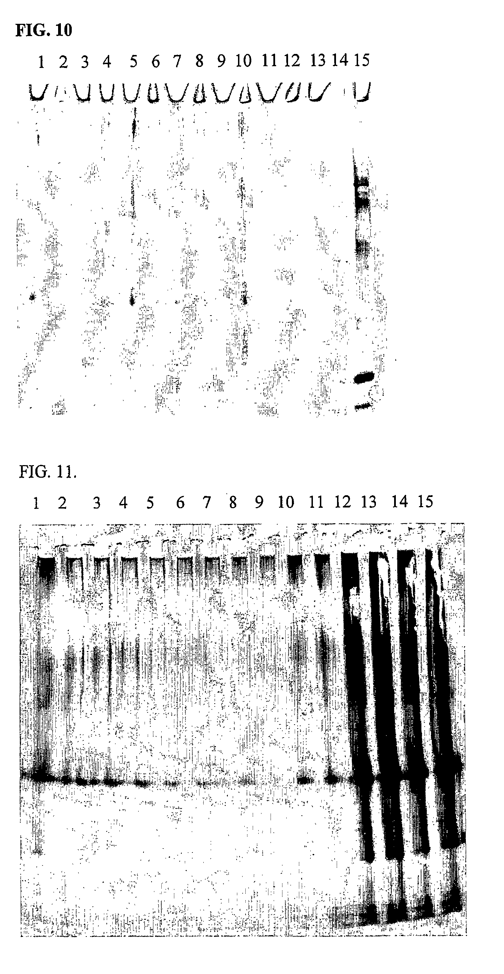

After insertion of the coated needles described in Example 1 into the gel, immediate withdraw of the needle was compared to a 1 min application into the 4-20 % tris-glycine Novex gel. Again, after electrophoresis a silver stain was applied to stain the HepB protein. The results are shown in FIG 10; the lanes correspond to: 1. HepB coated needle in lactose inserted and withdrawn after 1 min; 2. HepB coated needle in lactose inserted and withdrawn immediately; 3. empty; 4. HepB coated needle in sucrose inserted and withdrawn after 1 min; 5. HepB coated needle in sucrose inserted and withdrawn immediately; 6. empty; 7. HepB coated needle in raffinose inserted and withdrawn after 1 min; 8. HepB coated needle in raffinose inserted and withdrawn immediately; 9. empty; 10. HepB coated needle in trehalose inserted and withdrawn after 1 min; 11. HepB coated needle in trehalose inserted and withdrawn immediately; 12., 13., 14. empty; 15. MW markers (Biolabs).

### Example 3, Lyophilisation of needles coated with HepB at 444 µg/ml in high % of sucrose formulations.

From starting solutions of Hep B (888 µg/ml) and sucrose solution (at 60% w/v), a coating preparation was made resulting in Hep B at 444 µg/ml in 40% sucrose, in PBS. As for Example 1, needles are fixed on a standard rubber stopper used for lyophilisation. The needles were coated by plunging (2.5 cm deep) them either once or five times (with the needles allowed to dry between each coating step), into the liquid Hep B formulation. Needle and rubber stopper are placed in a regular lyophilisation vial, and submitted to a standard lyophilisation cycle. After lyophilisation, the vials were closed by pushing completely the stopper on the vial, so that the coated needles are kept in a closed vial during storage.

| Dipping | Sucrose | PO4 | NaCl | HbsAg |
|---|---|---|---|---|
| One time | 40% | 5 mM | 75 mM | 444 µg / ml |
| Five times | 40% | 5 mM | 75 mM | 444 µg / ml |

### Analysis & SDS-PAGE conditions of coated needles (after lyophilisation)

Dry coated needles of each formulation are applied directly on gel by stinging them (2 cm deep) inside the gel. No reducing treatment is applied. The gel is a 4-20 % tris-glycine Novex. After electrophoresis, silver stain is applied. The results for the five time dippings are shown in FIG 11, with the lanes corresponding to: 1. Hep B purified bulk 1 µg; 2. Hep B purified bulk 0.5 µg; 3. Hep B purified bulk 0.3 µg; 4. Hep B purified bulk 0.2 µg; 5. Hep B purified bulk 0.1 µg; 6. Hep B purified bulk 0.05 µg; 7. Hep B purified bulk 0.01 µg; 8/9/10/11 empty; 12/13/14/15 Needle lyophilised with formulation 40% sucrose 5 layers.

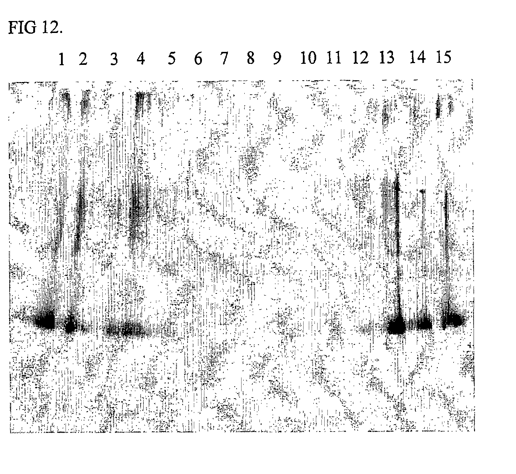

The results for the single dipping procedure are shown in FIG 12. with the lanes corresponding to: 1. Hep B purified bulk 1 µg; 2. Hep B purified bulk 0.5 µg; 3. Hep B purified bulk 0.3 µg; 4. Hep B purified bulk 0.2 µg; 5. Hep B purified bulk 0.1 µg; 6. Hep B purified bulk 0.05 µg; 7. Hep B purified bulk 0.01 µg; 8/9/10/11 empty; 12/13/14/15 Needle lyophilised with formulation 40% sucrose single layer.

Thus, using Hep B at 444µg/ml and sucrose at 40% solution, it is possible to coat more than 1 µg per needle and probably around 5µg deposit after 5 plunging operations.

## Claims

1. A pharmaceutical agent delivery device having at least one skin-piercing member comprising a solid biodegradable reservoir medium containing the pharmaceutical agent wherein the solid biodegradable reservoir medium is a polyol and wherein the pharmaceutical agent is a vaccine.

2. A pharmaceutical agent delivery device as claimed in claim 1, wherein the solid biodegradable reservoir medium containing the pharmaceutical agent is coated externally onto the at least one skin-piercing member.

3. A pharmaceutical agent delivery device as claimed in claim 2, wherein the polyol is a stabilising polyol.

4. A pharmaceutical agent delivery device as claimed in any one of claims 1 to 3 wherein the solid biodegradable reservoir medium is a sugar.

5. A pharmaceutical agent delivery device as claimed in claim 4 wherein the sugar is selected from lactose, sucrose, raffinose or trehalose.

6. A pharmaceutical agent delivery device as claimed in any one of claims 1 to 4 wherein the solid biodegradable reservoir medium forms a glass.

7. A pharmaceutical agent delivery device as claimed in any on of claims 1 to 6 wherein the solid biodegradable reservoir medium releases the pharmaceutical agent within 5 minutes after insertion of the skin-piercing member and solid biodegradable reservoir medium into the skin.

8. A pharmaceutical agent delivery device as claimed in any one of claims 1 to 7 wherein the skin piercing members are dimensioned to deliver the agent into the dermis.

9. A pharmaceutical agent delivery device as claimed in any one of claims 1 to 7 wherein the skin piercing members are dimensioned to deliver the agent into the epidermis.

10. A pharmaceutical agent delivery device as claimed in any one of claims 1 to 9 wherein the skin piercing members are microneedles or microblades.

11. A pharmaceutical agent delivery device as claimed in claim 1 to 10 wherein the vaccine comprises an antigen.

12. A pharmaceutical agent delivery device as claimed in claim 1 to 10 wherein the vaccine comprises nucleic acid encoding an antigen.

13. A process for the preparation of a pharmaceutical delivery device according to any of claims 1 to 10 comprising making a solution of said pharmaceutical agent and said reservoir medium, followed by dipping said at least one skin-piercing member into said solution, and allowing the solution to dry onto the skin-piercing member to form said solid biodegradable reservoir medium containing said pharmaceutical agent.

14. A skin patch for delivery of vaccines comprising an array of microblades or microneedles coated with a glassy sugar reservoir medium containing a vaccine antigen.

## Patentansprüche

1. Übertragungsvorrichtung für ein Pharmazeutikum mit wenigstens einem Haudurchbohrungselement, das ein festes biologisch abbaubares Reservoirmedium umfaßt, das das Pharmazeutikum enthält, worin das feste biologisch abbaubare Reservoirmedium ein Polyol ist und worin das Pharmazeutikum ein Impfstoff ist.

2. Übertragungsvorrichtung für ein Pharmazeutikum gemäß Anspruch 1, worin das feste biologisch abbaubare Reservoirmedium, das das Pharmazeutikum enthält, extern auf das wenigstens eine Hautdurchbohrungselement aufgetragen ist.

3. Übertragungsvorrichtung für ein Pharmazeutikum gemäß Anspruch 2, worin das Polyol ein stabilisierendes Polyol ist.

4. Übertragungsvorrichtung für ein Pharmazeutikum gemäß einem der Ansprüche 1 bis 3, worin das feste biologisch abbaubare Reservoirmedium ein Zucker ist.

5. Übertragungsvorrichtung für ein Pharmazeutikum gemäß Anspruch 4, worin der Zucker aus Lactose, Saccharose, Raffinose oder Trehalose ausgewählt ist.

6. Übertragungsvorrichtung für ein Pharmazeutikum gemäß einem der Ansprüche 1 bis 4, worin das feste biologisch abbaubare Reservoirmedium ein Glas bildet.

7. Übertragungsvorrichtung für ein Pharmazeutikum gemäß einem der Ansprüche 1 bis 6, worin das feste biologisch abbaubare Reservoirmedium das Pharmazeutikum innerhalb von 5 Minuten nach Einführen des Hautdurchbohrungselements und des festen biologisch abbaubaren Reservoirmediums in die Haut freisetzt.

8. Übertragungsvorrichtung für ein Pharmazeutikum gemäß einem der Ansprüche 1 bis 7, worin die Hautdurchbohrungselemente dimensioniert sind, um das Pharmazeutikum in die Dermis zu übertragen.

9. Übertragungsvorrichtung für ein Pharmazeutikum gemäß einem der Ansprüche 1 bis 7, worin die Hautdurchbohrungselemente dimensioniert sind, um das Pharmazeutikum in die Epidermis zu übertragen.

10. Übertragungsvorrichtung für ein Pharmazeutikum gemäß einem der Ansprüche 1 bis 9, worin die Hautdurchbohrungselemente Mikronadeln oder Mikroklingen sind.

11. Übertragungsvorrichtung für ein Pharmazeutikum gemäß Ansprüchen 1 bis 10, worin der Impfstoff ein Antigen umfaßt.

12. Übertragungsvorrichtung für ein Pharmazeutikum gemäß Anspruch 1 bis 10, worin der Impfstoff Nukleinsäure umfaßt, die ein Antigen codiert.

13. Verfahren zur Herstellung einer pharmazeutischen Übertragungsvorrichtung gemäß einem der Ansprüche 1 bis 10, umfassend das Herstellen einer Lösung aus dem Pharmazeutikum und dem Reservoirmedium, gefolgt von Eintauchen des wenigstens einen Hautdurchbohrungselements in die Lösung und Trocknenlassen der Lösung auf dem Hautdurchbohrungselement zur Bildung des festen biologisch abbaubaren Reservoirmediums, das das Pharmazeutikum enthält.

14. Hautpflaster zur Übertragung von Impfstoffen, umfassend eine Anordnung von Mikroklingen oder Mikronadeln, die mit einem glasartigen Zucker-Reservoirmedium beschichtet sind, das ein Impfstoffantigen enthält.

## Revendications

1. Dispositif de distribution d'agent pharmaceutique ayant au moins un élément perçant la peau, comprenant un milieu réservoir solide biodégradable contenant l'agent pharmaceutique, dans lequel le milieu réservoir solide biodégradable est un polyol et dans lequel l'agent pharmaceutique est un vaccin.

2. Dispositif de distribution d'agent pharmaceutique selon la revendication 1, dans lequel le milieu réservoir solide biodégradable contenant l'agent pharmaceutique est déposé extérieurement sur au moins un élément perçant la peau.

3. Dispositif de distribution d'agent pharmaceutique selon la revendication 2, dans lequel le polyol est un polyol stabilisateur.

4. Dispositif de distribution d'agent pharmaceutique selon l'une quelconque des revendications 1 à 3, dans lequel le milieu réservoir solide biodégradable est un sucre.

5. Dispositif de distribution d'agent pharmaceutique selon la revendication 4, dans lequel le sucre est choisi parmi le lactose, le saccharose, le raffinose ou le tréhalose.

6. Dispositif de distribution d'agent pharmaceutique selon l'une quelconque des revendications 1 à 4, dans lequel le milieu réservoir solide biodégradable forme un verre.

7. Dispositif de distribution d'agent pharmaceutique selon l'une quelconque des revendications 1 à 6, dans lequel le milieu réservoir solide biodégradable libère l'agent pharmaceutique dans les 5 minutes suivant l'insertion de l'élément perçant la peau et du milieu réservoir solide biodégradable dans la peau.

8. Dispositif de distribution d'agent pharmaceutique selon l'une quelconque des revendications 1 à 7, dans lequel les éléments perçant la peau sont dimensionnés de manière à distribuer l'agent dans le derme.

9. Dispositif de distribution d'agent pharmaceutique selon l'une quelconque des revendications 1 à 7, dans lequel les éléments perçant la peau sont dimensionnés de manière à distribuer l'agent dans l'épiderme.

10. Dispositif de distribution d'agent pharmaceutique selon l'une quelconque des revendications 1 à 9, dans lequel les éléments perçant la peau sont des micro-aiguilles ou des microlames.

11. Dispositif de distribution d'agent pharmaceutique selon la revendication 1 à 10, dans lequel le vaccin comprend un antigène.

12. Dispositif de distribution d'agent pharmaceutique selon la revendication 1 à 10, dans lequel le vaccin comprend l'acide nucléique codant un antigène.

13. Procédé de préparation d'un dispositif de distribution pharmaceutique selon l'une quelconque des revendications 1 à 10, comprenant la fabrication d'une solution dudit agent pharmaceutique et dudit milieu réservoir, suivie par l'immersion dudit au moins un élément perçant la peau dans ladite solution et le fait de laisser la solution sécher sur l'élément perçant la peau pour former ledit milieu réservoir solide biodégradable contenant ledit agent pharmaceutique.

14. Patch cutané pour la distribution de vaccins, comprenant un réseau de microlames ou de micro-aiguilles enduites d'un milieu réservoir de sucre vitrifié contenant un antigène vaccin.
